(19) Europäisches Patentamt
European Patent Office
Office européen des brevets

(11) EP 0 888 293 B1

(12) **EUROPEAN PATENT SPECIFICATION**

(45) Date of publication and mention of the grant of the patent:
**27.03.2002 Bulletin 2002/13**

(21) Application number: **96945113.7**

(22) Date of filing: **26.12.1996**

(51) Int Cl.[7]: **C07C 281/16**, A61K 31/155

(86) International application number:
**PCT/US96/20810**

(87) International publication number:
**WO 97/23447 (03.07.1997 Gazette 1997/29)**

(54) **N-ACYLAMINOALKYLHYDRAZINECARBOXIMIDAMIDES**

N-ACYLAMINOALKYLHYDRAZINECARBOXIMIDAMIDE

N-ACYLAMINOALKYLHYDRAZINECARBOXIMIDAMIDES

(84) Designated Contracting States:
**AT BE CH DE DK ES FI FR GB GR IE IT LI LU MC NL PT SE**
Designated Extension States:
**AL LT LV RO SI**

(30) Priority: **26.12.1995 US 9220 P**
**19.03.1996 US 618407**
**23.12.1996 US 771959**

(43) Date of publication of application:
**07.01.1999 Bulletin 1999/01**

(73) Proprietor: **ALTEON Inc.**
**Ramsey, New Jersey 07446 (US)**

(72) Inventors:
- **ULRICH, Peter, C.**
**Old Tappan, NJ 07675 (US)**
- **WAGLE, Dilip, R.**
**Valley Cottage, NY 10989 (US)**

(74) Representative: **Mercer, Christopher Paul**
**Carpmaels & Ransford**
**43, Bloomsbury Square**
**London WC1A 2RA (GB)**

(56) References cited:
**US-A- 4 758 583**

- **EUR. J. MED. CHEM. (1987), 22(6), 553-8 CODEN: EJMCA5;ISSN: 0223-5234, 1987, XP000579748 NAKASHIMA, KUNIO ET AL: "Methylglyoxal bis(guanylhydrazone) analogs. Multifunctional inhibitors for polyamine enzymes"**
- **J. ORG. CHEM. (1993), 58(16), 4331-8 CODEN: JOCEAH;ISSN: 0022-3263, 1993, XP000670003 WAGENAAR, FRANK L. ET AL: "Methodology for the preparation of N-guanidino-modified arginines and related derivatives"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 888 293 B1

## Description

The present invention relates generally to the aging of proteins resulting from their reaction with glucose and other reducing sugars, and more particularly to the inhibition of the reaction of nonenzymatically glycosylated proteins and the often resultant formation of advanced glycosylation (glycation) endproducts and cross-links.

The reaction between glucose and proteins has been known for some time. Its earliest manifestation was in the appearance of brown pigments during the cooking of food, which was identified by Maillard in 1912, who observed that glucose or other reducing sugars react with amino acids to form adducts that undergo a series of dehydrations and rearrangements to form stable brown pigments. Further studies have suggested that stored and heat treated foods undergo nonenzymatic browning as a result of the reaction between glucose and the polypeptide chain, and that the proteins are resultingly cross-linked and correspondingly exhibit decreased bioavailability.

This reaction between reducing sugars and food proteins was found to have its parallel *in vivo.* Thus, the nonenzymatic reaction between glucose and the free amino groups on proteins to form a stable, 1-deoxyketosyl adduct, known as the Amadori product, has been shown to occur with hemoglobin, wherein a rearrangement of the amino terminal of the beta-chain of hemoglobin by reaction with glucose, forms the adduct known as hemoglobin $A_{1c}$. The reaction has also been found to occur with a variety of other body proteins, such as lens crystallins, collagen and nerve proteins. See Bucala et al., "Advanced Glycosylation; Chemistry, Biology, and Implications for Diabetes and Aging" in Advances in Pharmacology, Vol. 23, pp. 1-34, Academic Press (1992).

Moreover, brown pigments with spectral and fluorescent properties similar to those of late-stage Maillard products have also been observed *in vivo* in association with several long-lived proteins, such as lens proteins and collagen from aged individuals. An age-related linear increase in pigment was observed in human dura collagen between the ages of 20 to 90 years. Interestingly, the aging of collagen can be mimicked *in vitro* by the cross-linking induced by glucose; and the capture of other proteins and the formation of adducts by collagen, also noted, is theorized to occur by a cross-linking reaction, and is believed to account for the observed accumulation of albumin and antibodies in kidney basement membrane.

In U.S. Patent 4,758,583, a method and associated agents were disclosed that served to inhibit the formation of advanced glycosylation endproducts by reacting with an early glycosylation product that results from the original reaction between the target protein and glucose. Accordingly, inhibition was postulated to take place as the reaction between the inhibitor and the early glycosylation product appeared to interrupt the subsequent reaction of the glycosylated protein with additional protein material to form the cross-linked late-stage product. One of the agents identified as an inhibitor was aminoguanidine, and the results of further testing have borne out its efficacy in this regard.

While the success that has been achieved with aminoguanidine and similar compounds is promising, a need continues to exist to identify and develop additional inhibitors that broaden the availability and perhaps the scope of this potential activity and its diagnostic and therapeutic utility.

In accordance with the present invention, agents, and compositions including pharmaceutical compositions containing said agents which are believed to inhibit the formation of advanced glycosylation endproducts in a number of target proteins existing in both animals and plant material. In particular, the invention relates to a compound having the structural formula:

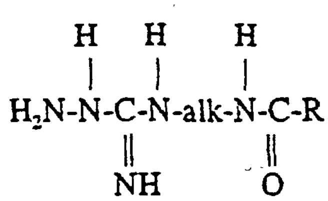

(I)

wherein alk is a straight or branched chain alkylene group containing from 2 to 8 carbon atoms, and R is a lower alkyl group containing from 1 to 6 carbon atoms; or a biologically or pharmaceutically acceptable acid addition salt thereof for inhibiting the advanted glycosylation of a target protein.

The lower alkyl groups referred to above preferably contain 1-6 carbon atoms and include methyl, ethyl, propyl, butyl, pentyl, hexyl, and the corresponding branched-chain isomers thereof. These groups are optionally substituted by one or more halo, hydroxy, amino or lower alkylamino groups.

The alkylene groups referred to herein contain from 2 to 8 carbon atoms and likewise can be straight or branched chain, and are thus exemplified by ethylene, propylene, butylene, pentylene, hexylene, heptylene, octylene, and their corresponding branched chain isomers.

The halo atoms in the above formula may be fluoro, chloro, bromo or iodo. The lower alkoxy groups contain

1-6, and preferably 1-3, carbon atoms and are illustrated by methoxy, ethoxy, n-propoxy, isopropoxy.

For the purposes of this invention equivalent to the compounds of formula (I) are the biologically and pharmaceutically acceptable acid addition salts thereof. Such acid addition salts may be derived from a variety of organic and inorganic acids such as sulfuric, phosphoric, hydrochloric, hydrobromic, sulfamic, citric, lactic, maleic, succinic, tartaric, cinnamic, acetic, benzoic, gluconic, ascorbic, methanesulfonic acids.

Of the compounds encompassed by Formula I, certain substituents are preferred. For instance, the compounds wherein alk is an ethylene group and R is a methyl group are preferred. In particular, the novel compound which is N-(2-acetamidoethyl)hydrazinecarboximidamide hydrochloride or hydrobromide exhibits a profile of activity which is particularly useful for the treatment of mammalian, especially human, patients.

Representative compounds of the present invention include:

N-(2-acetamidoethyl)hydrazinecarboximidamide hydrobromide;
N-(2-acetamidoethyl)hydrazinecarboximidamide hydrochloride;
N-(3-acetamidopropyl)hydrazinecarboximidamide hydrobromide; and
N-(4-acetamidobutyl)hydrazinecarboximidamide hydrobromide.

The above compounds are capable of inhibiting the formation of advanced glycosylation endproducts on target proteins. The cross-linking of the protein to form the advanced glycosylation endproduct contributes to the entrapment of other proteins and results in the development *in vivo* of conditions such as reduced elasticity and wrinkling of the skin, certain kidney diseases, atherosclerosis and osteoarthritis. Similarly, plant material that undergoes nonenzymatic browning deteriorates and, in the case of foodstuffs, become spoiled or toughened and, consequently, inedible. Thus, the compounds employed in accordance with this invention inhibit this late-stage Maillard effect and intervene in the deleterious changes described above.

The rationale of the present invention is to use agents which block the post-glycosylation step, *i.e.,* the formation of fluorescent chromophores, the presence of which chromophores is associated with, and leads to adverse sequelae of diabetes and aging. An ideal agent would prevent the formation of the chromophore and its associate cross-links of proteins to proteins and trapping of proteins on the other proteins, such as occurs in arteries and in the kidney.

The chemical nature of the early glycosylation products with which the compounds of the present invention are believed to react may vary, and accordingly the term "early glycosylation product(s)" as used herein is intended to include any and all such variations within its scope. For example, early glycosylation products with carbonyl moieties that are involved in the formation of advanced glycosylation endproducts, and that may be blocked by reaction with the compounds of the present invention, have been postulated. In one embodiment, it is envisioned that the early glycosylation product may comprise the reactive carbonyl moieties of Amadori products or their further condensation, dehydration and/or rearrangement products, which may condense to form advanced glycosylation endproducts. In another scenario, reactive carbonyl compounds, containing one or more carbonyl moieties (such as glycolaldehyde, glyceraldehyde or 3-deoxyglucosone) may form from the cleavage of Amadori or other early glycosylation endproducts, and by subsequent reactions with an amine or Amadori product, may form carbonyl containing advanced glycosylation products such as alkylformyl-glycosylpyrroles.

Several investigators have studied the mechanism of advanced glycosylation product formation. In vitro studies by Eble et al., (1983), "Nonenzymatic Glucosylation and Glucose-dependent Cross-linking of Protein", J. Biol. Chem., 258:9406-9412, concerned the cross-linking of glycosylated protein with nonglycosylated protein in the absence of glucose. Eble et al. sought to elucidate the mechanism of the Maillard reaction and accordingly conducted controlled initial glycosylation of RNAase as a model system, which was then examined under varying conditions. In one aspect, the glycosylated protein material was isolated and placed in a glucose-free environment and thereby observed to determine the extent of cross-linking.

Eble et al. thereby observed that cross-linking continued to occur not only with the glycosylated protein but with non-glycosylated proteins as well. One of the observations noted by Eble et al. was that the reaction between glycosylated protein and the protein material appeared to occur at the location on the protein chain of the amino acid lysine. Confirmatory experimentation conducted by Eble et al. in this connection demonstrated that free lysine would compete with the lysine on RNAase for the binding of glycosylated protein. Thus, it might be inferred from these data that lysine may serve as an inhibitor of advanced glycosylation; however, this conclusion and the underlying observations leading to it should be taken in the relatively limited context of the model system prepared and examined by Eble et al. Clearly, Eble et al. does not appreciate, nor is there a suggestion therein, of the discoveries that underlie the present invention, with respect to the inhibition of advanced glycosylation of proteins both *in vitro* and *in vivo.*

The experiments of Eble *et al.* do not suggest the reactive cleavage product mechanism or any other mechanism in the *in vivo* formation of advanced glycosylation endproducts in which glucose is always present. In fact, other investigators support this mechanism to explain the formation of advanced glycosylated endproducts *in vivo* (see for

example Hayase *et al, J. Biol. Chem.,* **263,** pp. 3758-3764 (1989); Sell and Monnier, *J. Biol. Chem.* **264,** pp. 21597-21602 (1989) Oimomi *et al., Agric. Biol. Chem.*, **53**(6):1727-1728 (1989); and *Diabetes Research and Clinical Practice,* **6**:311-313 (1989). Accordingly, the use of lysine as an inhibitor in the Eble *et al.* model system has no bearing upon the utility of the compounds of the present invention in the inhibition of advanced glycosylated endproducts formation in the presence of glucose *in vivo*, and the amelioration of complications of diabetes and aging.

The compositions useful in the present invention comprise or contain agents capable of reacting with the active carbonyl intermediate of an early glycosylation product. Suitable agents are the compounds of Formula I of the present invention.

In the food industry, sulfites were found years ago to inhibit the Maillard reaction and are commonly used in processed and stored foods. Recently, however, sulfites in food have been implicated in severe and even fatal reactions in asthmatics. As a consequence, the sulfite treatment of fresh fruits and vegetables has been banned. The mechanism for the allergic reaction is not known. Accordingly, the present compositions and agents offer a nontoxic alternative to sulfites in the treatment of foods in this manner.

As is apparent from a discussion of the environment of the present invention, the present compositions hold the promise for arresting the aging of key proteins both in animals and plants, and concomitantly, conferring both economic and medical benefits as a result thereof. In the instance of foodstuffs, the administration of the present composition holds the promise for retarding food spoilage thereby making foodstuffs of increased shelf life and greater availability to consumers. Replacement of currently-used preservatives, such as sulfur dioxide known to cause allergies and asthma in humans, with non-toxic, biocompatible compounds is a further advantage of the present invention.

The therapeutic implications relate to the arrest of the aging process which has, as indicated earlier, been identified in the aging of key proteins by advanced glycosylation and cross-linking. Thus, body proteins, and particularly structural body proteins, such as collagen, elastin, lens proteins, nerve proteins, kidney glomerular basement membranes and other extravascular matrix components would all benefit in their longevity and operation from the practice of the present invention. The present invention thus reduces the incidence of pathologies involving the entrapment of proteins by cross-linked target proteins, such as retinopathy, cataracts, diabetic kidney disease, glomerulosclerosis, peripheral vascular disease, arteriosclerosis obliterans, peripheral neuropathy, stroke, hypertension. atherosclerosis, osteoarthritis, periarticular rigidity, loss of elasticity and wrinkling of skin, stiffening of joints, glomerulonephritis, etc. Likewise, all of these conditions are in evidence in patients afflicted with diabetes mellitus. Thus, the compounds and compositions are relevant to treatment of the noted conditions in patients either of advanced age or those suffering from one of the mentioned pathologies.

Accordingly, in another aspect of the present invention, there is provided a compound of formula (I) or a biologically or pharmaceutically acceptable acid addition salt thereof for use in (i) improving the elasticity or reducing wrinkles of the skin, or treating or preventing (ii) hypertension, (iii) retinopathy, (iv) damage to lens proteins, (v) cataracts, (vi) peripheral neuropathy, or (vii) osteoarthritis.

In a further aspect of the present invention there is provided a compound of formula (I) or a biologically or pharmaceutically acceptable acid addition salt thereof for use in treating diabetes, or treating or preventing adverse sequalae of diabetes.

In a further aspect of the present invention there is provided a compound of formula (I) or a biologically or pharmaceutically acceptable acid addition salt thereof for use in treating or preventing kidney damage.

In a further aspect of the present invention there is provided a compound of formula (I) or a biologically or pharmaceutically acceptable acid addition salt thereof for use in treating or preventing blood vasculature.

In a further aspect of the present invention there is provided a pharmaceutical composition for use in (i) improving the elasticity or reducing wrinkles of the skin, treating (ii) diabetes or treating or preventing (iii) adverse sequalae of diabetes, (iv) kidney damage, (v) damage to blood vasculature, (vi) hypertension, (vii) retinopathy, (viii) damage to lens proteins, (ix) cataracts, (x) peripheral neuropathy, or (xi) osteoarthritis, comprising a pharmaceutically effective amount of a compound of formula (I) together with a carrier therefor.

Protein cross-linking through advanced glycosylation product formation can decrease solubility of structural proteins such as collagen in vessel walls and can also trap serum proteins, such as lipoproteins to the collagen. Also, this may result in increased permeability of the endothelium and consequently covalent trapping of extravasated plasma proteins in subendothelial matrix, and reduction in susceptibility of both plasma and matrix proteins to physiologic degradation by enzymes. For these reasons, the progressive occlusion of diabetic vessels induced by chronic hyperglycemia has been hypothesized to result from excessive formation of glucose-derived cross-links. Such diabetic microvascular changes and microvascular occlusion can be effectively prevented by chemical inhibition of advanced glycosylation product formation utilizing a composition of the invention.

Studies indicate that the development of chronic diabetic damage in target organs is primarily linked to hyperglycemia so that tight metabolic control would delay or even prevent end-organ damage. See Nicholls *et al., Lab. Invest.,* **60,** No. 4, p. 486 (1989), which discusses the effects of islet isografting and aminoguanidine in murine diabetic nephropathy. These studies further evidence that aminoguanidine diminishes aortic wall protein cross-linking in diabetic

rats and confirm earlier studies by Brownlee *et al., Science,* **232,** pp. 1629-1632 (1986) to this additional target organ of complication of diabetes. Also, an additional study showed the reduction of immunoglobulin trapping in the kidney by aminoguanidine (Brownlee *et al., Diabetes*, **35,** Suppl. 1, p. 42A (1986)).

Further evidence in the streptozotocin-diabetic rat model that aminoguanidine administration intervenes in the development of diabetic nephropathy was presented by Brownlee et al., 1988, *supra,* with regard to morphologic changes in the kidney which are hallmarks of diabetic renal disease. These investigators reported that the increased glomerular basement membrane thickness, a major structural abnormality characteristic of diabetic renal disease, was prevented with aminoguanidine.

Taken together, these data strongly suggest that inhibition of the formation of advanced glycosylation endproducts (AGEs), by the teaching of the present invention, may prevent late, as well as early, structural lesions due to diabetes, as well as changes during aging caused by the formation of AGEs.

Diabetes-induced changes in the deformability of red blood cells, leading to more rigid cell membranes, is another manifestation of cross-linking and aminoguanidine has been shown to prevent it *in vivo.* In such studies, New Zealand White rabbits, with induced, long-term diabetes are used to study the effects of a test compound on red blood cell (RBC) deformability (df). The test compound is administered at a rate of 100 mg/kg by oral gavage to diabetic rabbits.

A further consequence of diabetes is the hyperglycemia-induced matrix bone differentiation resulting in decreased bone formation usually associated with chronic diabetes. In animal models, diabetes reduces matrix-induced bone differentiation by 70%.

Aminoguanidine, although a promising therapeutic agent, exhibits other pharmacological activities which, in certain circumstances, may limit its therapeutic usefulness in certain patient populations. Thus, the search has continued for an inhibitor of the formation of advanced glycosylation endproducts which is selective for this activity without significant effects on the enzymes diamino oxidase (DAO) and the inducible form of nitric oxide synthase (iNOS). The instant invention identifies the novel compound N-(2-acetamidoethyl)hydrazinecarboximidamide and its various salt forms, especially the hydrochloride, as uniquely being a selective inhibitor of the formation of advanced glycosylation endproducts without appreciable effects upon the diamino oxidase enzyme or nitric oxide formation. Thus, the present invention provides compositions for the inhibition of the formation of advanced glycosylation endproducts without concommitant inhibition of diamino oxidase and nitric oxide formation which comprise N-(2-acetamidoethyl)hydrazinecarboximidamide and its various salt forms.

In the instance where the compositions of the present invention are utilized for *in vivo* or therapeutic purposes, it may be noted that the compounds or agents used therein are biocompatible. Pharmaceutical compositions may be prepared with a therapeutically effective quantity of the agents or compounds of the present invention and may include a pharmaceutically acceptable carrier, selected from known materials utilized for this purpose. Such compositions may be prepared in a variety of forms, depending on the method of administration. Also, various pharmaceutically acceptable addition salts of the compounds of Formula I may be utilized.

A liquid form would be utilized in the instance where administration is by intravenous, intramuscular or intraperitoneal injection. When appropriate, solid dosage forms such as tablets, capsules, or liquid dosage formulations such as solutions and suspensions. etc., may be prepared for oral administration. For topical or dermal application to the skin or eye, a solution, a lotion or ointment may be formulated with the agent in a suitable vehicle such as water, ethanol, propylene glycol, perhaps including a carrier to aid in penetration into the skin or eye. For example, a topical preparation could include up to about 10% of the compound of Formula I. Other suitable forms for administration to other body tissues are also contemplated.

In the instance where the method has therapeutic application, the animal host intended for treatment may have administered to it a quantity of one or more of the agents, in a suitable pharmaceutical form. Administration may be accomplished by known techniques, such as oral, topical and parenteral techniques such as intradermal, subcutaneous, intravenous or intraperitoneal injection, as well as by other conventional means. Administration of the agents may take place over an extended period of time at a dosage level of, for example, up to about 30 mg/kg.

The invention also extends to compounds according to Formula (I) for use in inhibiting the discoloration of teeth resulting from nonenzymatic browning in the oral cavity.

The nonenzymatic browning reaction which occurs in the oral cavity results in the discoloration of teeth. Presently used anti-plaque agents accelerate this nonenzymatic browning reaction and further the staining of the teeth. Recently, a class of cationic anti-microbial agents with remarkable anti-plaque properties have been formulated in oral rinses for regular use to kill bacteria in the mouth. These agents, the cationic antiseptics, include such agents as alexidine, cetyl pyridinium chloride, chlorhexidine gluconate, hexetidine, and benzalkonium chloride.

Tooth staining by chlorhexidine and other anti-plaque agents apparently results from the enhancement of the Maillard reaction. Nordbo, *J. Dent. Res.,* **58,** p. 1429 (1979) reported that chlorhexidine and benzalkonium chloride catalyze browning reactions *in vitro.* Chlorhexidine added to mixtures containing a sugar derivative and a source of amino groups underwent increased color formation, attributed to the Maillard reaction. It is also known that use of

chlorhexidine results in an increased dental pellicle. Nordbo proposed that chlorhexidine resulted in tooth staining in two ways: first, by increasing formation of pellicle which contains more amino groups, and secondly, by catalysis of the Maillard reaction leading to colored products.

In accordance with this method, the compounds of Formula I are formulated into compositions adapted for use in the oral cavity. Particularly suitable formulations are oral rinses and toothpastes incorporating the active agent.

In the practice of this invention, conventional formulating techniques are utilized with nontoxic, pharmaceutically acceptable carriers typically utilized in the amounts and combinations that are well-known for the formulation of such oral rinses and toothpastes.

The agent of Formula I is formulated in compositions in an amount effective to inhibit the formation of advanced glycosylation endproducts. This amount will, of course, vary with the particular agent being utilized and the particular dosage form, but typically is in the range of 0.01% to 1.0%, by weight, of the particular formulation.

The compounds of formula I can be prepared according to the methods described in Nakashima *et al., Eur. J. Med. Chem.*, **22** 553-558 (1987). This reference describes, at page 554, the preparation of the hydrochloride salts of two compounds of formula I, *i.e.,*
N-(3-acetamidopropyl)hydrazinecarboximidamide (also known as 1-amino-3-acetamidopropylguanidine); and N-(4-acetamidobutyl)hydrazinecarboximidamide (also known as (1-amino-3-acetamidobutylguanidine).

One method of synthesis for the compounds of the instant invention is as shown in Scheme I below.

Scheme I

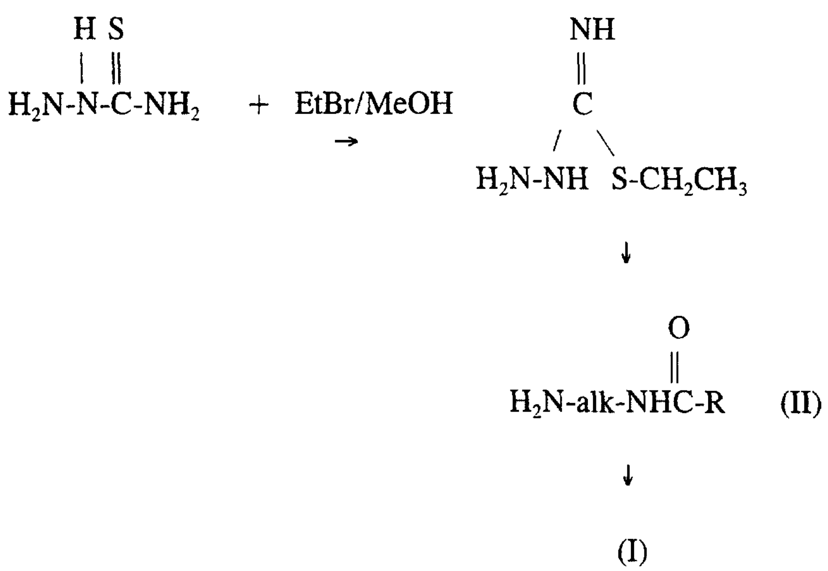


In this reaction scheme, thiosemicarbazide, dissolved in methanol, or another suitable polar solvent, is reacted with ethyl bromide and heated to reflux temperatures for a period of about 2 to 12 hours. Cooling to ambient temperatures and addition of t-butylmethyl ether to the methanolic solution resulted in precipitation of the S-ethylthiosemicarbazide hydrobromide.

Then, to the S-ethylthiosemicarbazide hydrobromide is added the appropriate N-acylalkylenediamine of formula II. After heating to reflux temperatures for a period of about 2 to 12 hours, and cooling, the desired N-acylaminoalkylhydrazinecarboximidamide of formula I is isolated, and, if desired, purified by recrystallization from a suitable solvent such as a mixture of isopropanol and t-butylmethyl ether.

The various acid addition salts of the compounds of formula I are typically prepared from the corresponding hydrobromide by neutralization of the hydrobromide with a strong base, such as sodium hydroxide, followed by treatment with a strong acid to give the corresponding salt. Alternatively, the hydrobromide salt can be dissolved in methanol, and treated with gaseous hydrogen chloride to afford the corresponding hydrochloride salt.

A further method of preparing the compounds of the instant invention involves their preparation as shown in the Scheme II below:

Scheme II

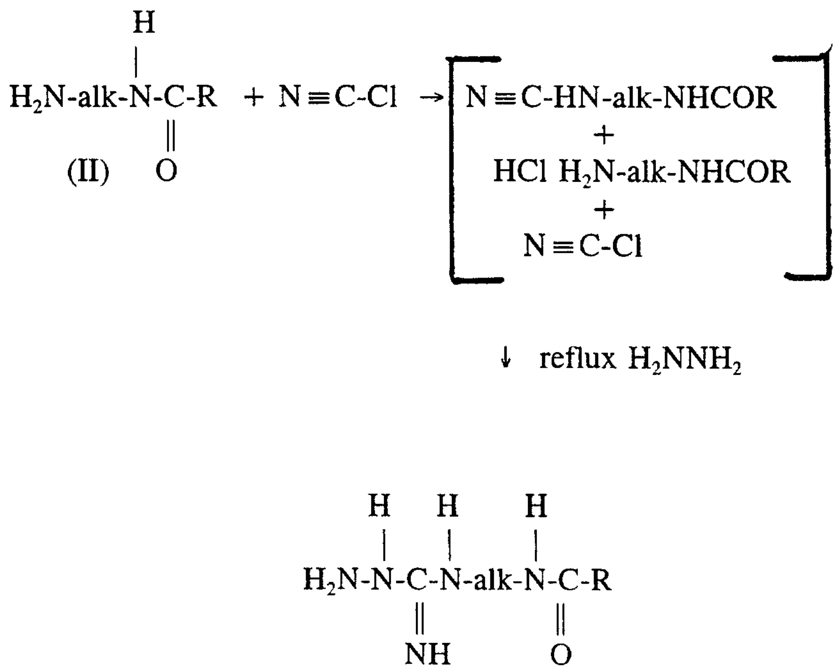


(I)

In this reaction Scheme II, cyanogen chloride is reacted with the appropriate N-acylalkylenediamine of formula II to afford a mixture of unisolated intermediates . These unisolated intermediates, shown in the reaction Scheme II above in the square brackets, are then refluxed with anyhydrous hydrazine in a polar solvent for a period of about 1 to about 15 hours.

Suitable polar solvents include alcohols such as 2-propanol and ethanol. The reaction temperature for the hydrazine addition step will, of course, depend upon, and vary with, the nature of the particular solvent chosen.

The following examples are illustrative of the invention.

EXAMPLE 1

**A. S-Ethylthiosemicarbazide hydrobromide.**

Thiosemicarbazide (218.4g, 2.4 mol) was taken up in ethanol (1L). To the mixture was added ethyl bromide (306.6g, 2.8 mol) and the mixture was slowly heated to reflux. As the reaction proceeded, all solids dissolved into solution in 7 hours. The solution was refluxed for two more hours and was cooled to the ambient temperature. tert-Butylmethyl ether (700 mL) was added to the ethanolic solution and kept in the refrigerator overnight. White crystals were collected in two crops (418g, 87%); mp 123-5°C. $^1$H-NMR($D_2O$) 3.18 (2H, dd), 1.39 (3H, t). $^{13}$C-NMR ($D_2O$) 169.6, 25.7, 13.8 ppm.

**B. N-(2-acetamidoethyl)hydrazine carboximidamide hydrobromide.** To a solution of S-ethylthiosemicarbazide hydrobromide (100 g, 0.5 mol) in 750 mL of ethanol was added N-acetyl-ethylenediamine (51 g, 0.5 mol). The mixture was refluxed for 8 hours under the system connected to four bleach traps. Upon cooling the mixture, a white crystal formed and was filtered off. The solution was evaporated to a red oil under a vaccum system connected to bleach traps. The oil slowly crystalized in 2-propanol/tBuOMe to give white crystals (39.5 g, 33%) of the title compound in three crops; mp 127-8° C. $^1$H-NMR ($D_2O$) 3.36 (4H, t), 2.00 (3H, S). $^{13}$C-NMR ($D_2O$) 175.0, 158.4, 40.6, 38.5, 22.3 ppm.

EXAMPLE 2

**N-(2-acetamidoethyl)hydrazine carboximidamide hydrochloride**

N-(2-acetamidoethyl)hydrazine carboximidamide hydrobromide (5g, 20.8 mmol) was dissolved in 50 mL of water. Amberlite IRA-400 (OH) (50 g from Aldrich) was loaded on a column (2.5 cm dia. x 45 cm) with a fritted glass. The resin was eluted with 200 mL of IN NaOH and then with distilled water until pH 7. The aqueous hydrobromide solution was loaded on the resin and eluted dropwise. More water was eluted until the eluant became neutral. All

aqueous fractions with positive ninhydrin test were combined and 10 mL of 6 N HCl was added. Water was removed *in vacuo* to give a colorless oil, which subsequently was recrytallized from 2-propanol to yield 3.82 g (94%); mp 127-8 °C. $^1$H-NMR ($D_2O$) 3.36 (4H, dt), 1.99 (3H, s). $^{13}$C-NMR ($D_2O$) 174.9, 158.2, 40.5, 38.4, 22.0 ppm.

EXAMPLE 3

**N-(2-acetamidoethyl)hydrazine carboximidamide hydrochloride.**

The hydrobromide salt N-(2-acetamidoethyl)hydrazine carboximidamide hydrobromide (5 g, 20.8 mmol) was dissolved in 100 mL of methanol. To the stirred solution was passed HCl gas for 15 minutes in an ice bath. After passing HCl gas, the solution was stirred for 2 hours at room temperature and 200 mL of tBuOMe was added. After 24 hours, an oil seperated on the bottom of the flask. The liquid was decanted from the oil. The oil crystallized from 2-propanol/ tBuOMe to give 3.3 g (82%) of white crystals which has the same physical properties with crystals from Method A.

EXAMPLE 4

In reaction sequence essentially as described in paragraphs A and B of Example 1, the appropriate starting materials are utilized to prepare the following compounds of formula I:

N-(3-acetamidopropyl)hydrazinecarboximidamide hydrobromide; and
N-(4-acetamidobutyl)hydrazinecarboximidamide hydrobromide.

EXAMPLE 5

**N-(2-acetamidoethyl)hydrazinecarboximidamide hydrochloride.**

The following process must be carried out in a well-ventilated hood. An outlet of a 2 L three-neck flask, whose weight was earlier measured, is connected to a 6N KOH trap and the whole flask is cooled to -70°C by immersing in a dry ice-acetone bath. To the flask is purged cyanogen chloride gas from a lecture bottle whose weight change was monitored on a balance. Sixty-two grams of cyanogen chloride (1 mol) is collected. To the liquified gas is then added cold 2-propanol (500 mL) and a mechanical stirrer apparatus. With vigorous stirring, a solution of N-acethylethylenediamine (102 g, 1 mol) in 2-propanol (500 mL) is added dropwise such that the internal temperature does not exceed 5°C. After addition is completed, the mixture is stirred for an hour at 5°C and anhydrous hydrazine (28.2mL, 0.9 mol) is added slowly. The mixture is stirred for an hour at 5°C, an additional hour at room temperature, and then refluxed for a further 15 hours. During the last hour of reflux, the volume of the reaction mixture is reduced by half by distillation removal of the solvent. Upon cooling, white cubic crystals slowly form. Crystals are then collected and dried to give the title compound.

EXAMPLE 6

The following method was used to evaluate the ability of the compounds of the present invention to inhibit the cross-linking of glycated bovine serum albumin (AGE-BSA) to the rat tail tendon collagen coated 96-well plate.

The AGE-BSA was prepared by incubating BSA at a concentration of 200 mg per ml with 200 mM glucose in 0.4M sodium phosphate buffer, pH 7.4 at 37°C for 12 weeks. The glycated BSA was then extensively dialyzed against phosphate buffer solution (PAS) for 48 hours with additional 5 times buffer exchanges. The rat tail tendon collagen coated plate was blocked first with 300 µl of superbloc blocking buffer (Pierce #37515X) for one hour. The blocking solution was removed from the wells by washing the plate twice with PAS-Tween 20 solution (0.05% Tween 20) using a NUNC-multiprobe or Dynatech ELISA-plate washer. Cross-linking of AGE-BSA (1 to 10 µg per well depending on the batch of AGE-BSA) to rat tail tendon collagen coated plate was performed with and without the testing compound dissolved in PAS buffer at pH 7.4 at the desired concentrations by the addition of 50 µl each of the AGE-BSA diluted in PAS or in the testing compound at 37°C for 4 hours. The unbrowned BSA in PAS buffer with or without testing compound were added to the separate wells as the blanks. The uncross-linked AGE-BSA was then removed by washing the wells three times with PAS-Tween buffer. The cross-linked AGE-BSA to the tail tendon coated plate was then quantitated by the polyclonal antibody raised against AGE-RNase. After a one-hour incubation period, AGE antibody was removed by washing 4 times with PAS-Tween.

The bound AGE antibody was then detected with the addition of horseradish peroxidase-conjugated secondary antibody, e.g., goat anti-rabbit immunoglobulin and incubation for 30 minutes. The substrate of 2,2-azino-di(3-ethylbenzthiazoline sulfonic acid) (ABTS chromogen) (Zymed #00-2011) was added. The reaction was allowed for an additional 15 minutes and the absorbance was read at 410 nm in a Dynatech plate reader.

The % inhibition of each test compound was calculated as follows.

% inhibition =

{[Optical density (without compound) - optical density (with compound)]/optical

density (without compound)]} 100%

The $IC_{50}$ relative inhibition by various test compounds at 10 mM is as follows:

| Test Compound | $IC_{50}$ |
|---|---|
| N-(2-acetamidoethyl)hydrazine-carboximidamide hydrobromide | 2.6 mM |
| N-(2-acetamidoethyl)hydrazine-carboximidamide hydrochloride | 3.6 mM |
| N-(3-acetamidopropyl)hydrazine-carboximidamide hydrobromide | 12.2 mM |
| Aminoguanidine hydrochloride | 12.0 mM |

The above experiment suggests that the compounds of formula I are more potent inhibitors of the formation of advanced glycosylation so that this type of drug therapy has benefits in reducing the pathology associated with the advanced glycosylation of proteins and the formation of cross-links between proteins and other macromolecules. Drug therapy may be used to prevent the increased trapping and cross-linking of proteins that occurs in diabetes and aging which leads to sequelae such as retinal damage, and extra-vascularly, damage to tendons, ligaments and other joints.

EXAMPLE 7

The following method was used to evaluate the ability of the compounds of the present invention to inhibit the cross-linking of N-acetyl glycyl-lysine methyl ester in the presence of ribose.

Materials:

N-acetylglycyllysine methyl ester (DP in formula below)
Ribose (R in formula below)
Test compounds (C in formula below)

Reagents:

0.5 M sodium phosphate buffer pH 7.4
N-acetylglycyllysine methyl ester in 0.5M sodium phosphate buffer, pH 7.4
Ribose: 800 mM
Test compounds dissolved in the above buffer and the pH is adjusted to 7.4, if necessary

Procedure:

Reaction mixtures are prepared as follows:

| | | | |
|---|---|---|---|
| 80 mg/ml N-acetylglycyllysine methyl ester/buffer | 0.1 | 0.1 | - |
| ribose | 0.1 | 0.1 | 0.1 |
| test compound | - | 0.1 | 0.1 |

(continued)

| | | | |
|---|---|---|---|
| buffer | 0.2 | 0.1 | 0.2 |

and incubated at 37°C for 16-24 hours. At the end of the incubation period, the fluorescence is read using an excitation wavelength of 350 nm and emission wavelength of 400 nm. The inhibition of the cross-linking is calculated from the decrease in the fluorescence in the presence of the test compounds according to the formula:

Inhibition (%) =

100 x [DPRC fluorescence - RC fluorescence]/DPR fluorescence

The Inhibition by various test compounds ($IC_{50}$) is as follows:

| Test compound | $IC_{50}$mM |
|---|---|
| N-(2-acetamidoethyl)hydrazine-carboximidamide hydrobromide | 11.46 |
| N-(2-acetamidoethyl)hydrazine-carboximidamide hydrochloride | 10.64 |
| N-(3-acetamidopropyl)hydrazine-carboximidamide hydrochloride | > 10.0 |
| Aminoguanidine hydrochloride | 10.0 |

The above experiment suggests that the compounds of formula I possess inhibitory activity in the formation of advanced glycosylation endproducts and this type of drug therapy will reduce the pathology associated with the advanced glycosylation of proteins and the formation of cross-links between proteins and other macromolecules. Drug therapy may be used to prevent the increased trapping and cross-linking of proteins that occurs in diabetes and aging which leads to sequelae such as retinal damage, and extra-vascularly, damage to tendons, ligaments and other joints.

EXAMPLE 8

To determine the effects of test compounds on the enzyme diamino oxidase the following assay procedure is utilized. This assay is a colorimetric kinetic enzyme assay based on the coversion of amine substrate to the corresponding aldehyde product which then reacts with reagent. The concentrations of enzyme and substrate used in this procedure ensure the reaction is at saturating conditions and linear for the time of incubation at room temperature.

Materials:

sodium phosphate buffer, 0.1 M, pH = 7.4
putrescine (Sigma), 0.03 M (4.8 mg/ml)
diamine oxidase (Sigma), 3 U/ml (50 mg/ml)
o-aminobenzaldehyde (Sigma), (0.5 mg/ml)
96-well plate, plate reader w/absorbance filter at 450 nm

Procedure:

The reaction is conducted at room temperature. All reaction reagents and test compound dilution should be made up fresh in buffer, with the exception of the sodium phosphate buffer which can be stored at 4°C for one month. Once prepared, the solutions can be maintained at room temperature. Putrescine and diamine oxidase are readily soluble in buffer. The *o*-aminobenzaldehyde (o-bzld) is light sensitive and requires slight heating at 37°C. This is done in a tube covered with alumninum foil. In addition, this reagent will begin to lose its activity following opening of the container due to oxidation so that it is necessary to work quickly with it. After warming up the container to room temperature, weigh out the necessary amount.

1. Pipet appropriate volumne of buffer to each well.
2. Add 50 µl of test compound to the appropriate wellls. Add 50 µl of diamino oxidase (DAO) enzyme solution to the test and total wells. Allow the compound to interact with the enzyme for a fifteen minute pre-incubation step.
3. Blank the spectrophotometer to air. With a repeating pipetor, add 50 ul of the o-aminobenzaldehyde solution to all the wells. Afterwards, add 50 ul of the putrescine substate solution to the test and total wells. Substrate is not added to the blank wells. The initail absorbance is read at 450 nm in the automatic plate reater. The reaction is then allowed to occur for 37 minutes, and then final absorbance is read.

Volumes:

| | BLANK(µl) | TOTAL(µl) | TEST(µl) |
|---|---|---|---|
| buffer | 200 | 150 | 100 |
| DAO | 50 | 50 | 50 |
| o-bzld | 50 | 50 | 50 |
| putrescine | 0 | 50 | 50 |
| compound | 0 | 0 | 50 |

D. Calculations:

The initial absorbance value for each test and blank well serves as the blank for that well. This allows for correction of the optical density which may arise from any interfering absorbance arising from the compound. The initial absorbance for the blank well is used as an index to determine if the titration of any compound is acceptable if any highly abnormal pattern of absorbance is seen upon dilution of a test compound.
Values for the initial absorbance determinations are subtracted from the final absorbance values. The results are expressed as a percentage of the total enzyme activity in the absence of any test compound:

$$TEST_{FINAL} - TEST_{INITIAL}/TOTAL_{FINAL} - TOTAL_{INITIAL}) \times 100$$

A semi-logarithmic plot of concentration versus percent total will allow for calculating $IC_{50}$ values. Use of a software program such as INPLOT is recommended.
When representative compounds of this invention and aminoguanidine were tested in this assay, the following results were obtained:

| Test compound | $IC_{50}$mM |
|---|---|
| N-(2-acetamidoethyl)hydrazine-carboximidamide hydrochloride | 6,858 |
| N-(3-acetamidopropyl)hydrazine-carboximidamide hydrochloride | 3,140 |
| Aminoguanidine hydrochloride | 21 |

EXAMPLE 8

To determine the effects of test compounds on the enzyme inducible nitric oxide synthase, and the resultant formation of nitric oxide, the following assay procedure is utilized.
Nitric oxide (NO) production by macrophage cells (RAW 264.7) is induced with lipopolysaccharride, and various test compounds are added to assess inhibition by the test compounds.
The cells are washed, counted and diluted to a concentration of $1.0 \times 10^6$ cells/ml. Then 100 ul cell/well are aliquoted to each well in a 96 well tissue culture plate to give a final concentraion of $1.0 \times 10^5$ cells/ml. The assay is set up in triplicate wells in order to harvest sufficient supernatant to be tested. The cells are incubated at 37°C oven for two hours, and then the test compound added thereto. Incubation is continued for a further three hours at 37°C, after which the lipopolysaccharide is added. Incubation is then continued overnight at 37°C, and then, to a 300 µl sample is added 400 µl of 1 % sulfanilamide in 4N HCl and 100 µl 6N HCl. This mixture is incubated for ten minutes

at room temperature, and then 300 µl of 1% N-(1-napthyl)ethylenediamine dihydrochloride in methanol is added. The optical density is read after each addition at 540 nm.

When representative compounds of this invention and aminoguanidine were tested in this assay, the following results were obtained:

| Test compound | % inhibition of NO release |
|---|---|
| N-(2-acetamidoethyl)hydrazine-carboximidamide hydrobromide | 20 |
| N-(2-acetamidoethyl)hydrazine-carboximidamide hydrochloride | 15 |
| N-(3 -acetamidopropyl)hydrazine-carboximidamide hydrochloride | 33 |
| Aminoguanidine hydrochloride | 44 |

EXAMPLE 10

| Tablet | mg/tablet |
|---|---|
| Compound of Formula I | 50 |
| Starch | 50 |
| Mannitol | 75 |
| Magnesium stearate | 2 |
| Stearic acid | 5 |

The compound, a portion of the starch and the lactose are combined and wet granulated with starch paste. The wet granulation is placed on trays and allowed to dry overnight at a temperature of 45°C. The dried granulation is comminuted in a comminutor to a particle size of approximately 20 mesh. Magnesium stearate, stearic acid and the balance of the starch are added and the entire mix blended prior to compression on a suitable tablet press. The tablets are compressed at a weight of 232 mg. using a 11/32" punch with a hardness of 4 kg. These tablets will disintegrate within a half hour according to the method described in USP XVI.

<u>EXAMPLE 11</u>

| Lotion | mg/g |
|---|---|
| Compound of Formula I | 1.0 |
| Ethyl alcohol | 400.0 |
| Polyethylene glycol 400 | 300.0 |
| Hydroxypropyl cellulose | 5.0 |
| Propylene glycol | to make 1.0 g |

<u>EXAMPLE 12</u>

To further study the ability of inhibitors of nonenzymatic browning to prevent the discoloration of protein on a surface, such as that which occurs on the tooth surface, the following surface browning experiment is performed. As a substitute for a pellicle-covered tooth surface, unexposed and developed photographic paper is used to provide a fixed protein (gelatin, i.e., collagen) surface on a paper backing. Five millimeter circles are punched and immersed for one week at 50°C in a solution of 100 mM glucose-6-phosphate in a 0.5 M phosphate buffer, pH 7.4, containing 3 mM sodium azide. Glucose-6-phosphate is a sugar capable of participating in nonenzymatic browning at a more rapid rate than glucose. In addition to the glucose-6-phosphate, chlorhexidine and/or a compound of Formula I are included. After incubation, the gelatin/paper disks are rinsed with water, observed for brown color, and photographed.

Incubation of the disks in glucose-6-phosphate alone shows slight brown color versus disks soaked in buffer

alone. Inclusion of chlorhexidine (in the form of Peridex® at a final concentration of 0.04% chlorhexidine) shows significant browning. Addition of a compound of Formula I to the chlorhexidine completely inhibits browning of the gelatin, as does inclusion of a compound of Formula I in the absence of chlorhexidine.

The slight brown color formed by the action of glucose-6-phosphate on the gelatin surface alone and its prevention by a compound of Formula I demonstrates the utility of the present invention in preventing nonenzymatic browning of tooth surfaces. The enhanced browning in the presence of chlorhexidine and its prevention with a compound of Formula I demonstrates the utility of the present invention in preventing the anti-plaque agent-enhanced nonenzymatic browning which occurs with chlorhexidine.

EXAMPLE 13

| Oral Rinse | % |
|---|---|
| Compound of Formula I: | 1.4 |
| Chlorhexidine gluconate | 0.12 |
| Ethanol | 11.6 |
| Sodium saccharin | 0.15 |
| FD&C Blue No. 1 | 0.001 |
| Peppermint Oil | 0.5 |
| Glycerine | 10.0 |
| Tween 60 | 0.3 |
| Water to | 100 |

EXAMPLE 14

| Toothpaste | % |
|---|---|
| Compound of Formula I: | 5.5 |
| Sorbitol, 70% in water | 25 |
| Sodium saccharin | 0.15 |
| Sodium lauryl sulfate | 1.75 |
| Carbopol 934, 6% dispersion in | 15 |
| Oil of Spearmint | 1.0 |
| Sodium hydroxide, 50% in water | 0.76 |
| Dibasic calcium phosphate dihydrate | 45 |
| Water to | 100 |

**Claims**

**1.** A compound of the formula (I)

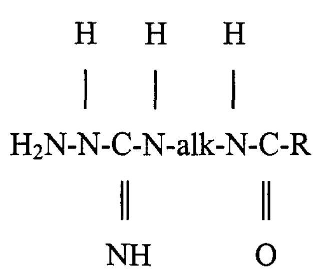

(I)

wherein

alk is a straight or branched chain alkylene group containing from 2 to 8 carbon atoms, and
R is a lower alkyl group containing from 1 to 6 carbon atoms;
or
a biologically or pharmaceutically acceptable acid addition salt thereof for inhibiting the advanced glycosylation of a target protein.

2. A compound as defined in formula (I) or a biologically or pharmaceutically acceptable acid addition salt thereof for use in (i) improving the elasticity or reducing wrinkles of the skin, or treating or preventing (ii) hypertension, (iii) retinopathy, (iv) damage to lens proteins, (v) cataracts, (vi) peripheral neuropathy, or (vii) osteoarthritis.

3. A compound as defined in formula (I) or a biologically or pharmaceutically acceptable acid addition salt thereof for use in treating diabetes, or treating or preventing adverse sequalae of diabetes.

4. A compound as defined in formula (I) or a biologically or pharmaceutically acceptable acid addition salt thereof for use in treating or preventing kidney damage.

5. A compound as defined in formula (I) or a biologically or pharmaceutically acceptable acid addition salt thereof for use in treating or preventing blood vasculature.

6. The compound of any one of claims 1-5 wherein R is a methyl group.

7. The compound of any one of claims 1-5 which is:

   N-(2-acetamidoethyl)hydrazinecarboximidamide hydrobromide;
   N-(2-acetamidoethyl)hydrazinecarboximidamide hydrochloride;
   N-(3-acetamidopropyl)hydrazinecarboximidamide hydrobromide; or

   another pharmaceutically acceptable salt thereof.

8. The compound of Claim 7 which is

   N-(2-acetamidoethyl)hydrazinecarboximidamide hydrochloride or
   N-(2-acetamidoethyl)hydrazinecarboximidamide hydrochloride hydrobromide

   for use in treating an animal to inhibit the formation of advanced glycosylation endproducts of a target protein within said animal, without concomitant effects upon the enzymes diaminooxidase and inducible nitric oxide synthase.

9. A pharmaceutical composition for administration to an animal to inhibit the advanced glycosylation of a target protein within said animal, comprising a pharmaceutically effective amount of a compound as defined in any one of Claims 1 to 8 together with a carrier therefor.

10. The pharmaceutical composition of Claim 9, for use in treating an animal to inhibit the formation of advanced glycosylation endproducts of a target protein within said animal.

11. A pharmaceutical composition for use in (i) improving the elasticity or reducing wrinkles of the skin, treating (ii) diabetes or treating or preventing (iii) adverse sequalae of diabetes, (iv) kidney damage, (v) damage to blood vasculature, (vi) hypertension, (vii) retinopathy, (viii) damage to lens proteins, (ix) cataracts, (x) peripheral neuropathy, or (xi) osteoarthritis, comprising a pharmaceutically effective amount of a compound as defined in any one of Claims 1 to 8 together with a carrier therefor.

12. A compound according to any one of Claims 1 to 8 or a composition according to Claim 9 for use in inhibiting the discoloration of teeth resulting from non-enzymatic browning in the oral cavity.

## Patentansprüche

1. Verbindung mit der Formel (I)

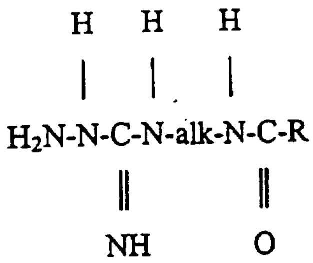

(I)

wobei alk ein geradkettiger oder verzweigtkettiger Aklylenrest, enthaltend 2 bis 8 Kohlenstoffatome, ist und R ein Niederalkylrest, enthaltend 1 bis 6 Kohlenstoffatome, ist, oder ein biologisch oder pharmazeutisch verträgliches Säureadditionssalz davon zur Inhibierung der fortschreitenden Glykosilierung eines Zielproteins.

2. Verbindung wie in Formel (I) definiert oder ein biologisch oder pharmazeutisch verträgliches Säureadditionssalz davon zur Verwendung für (i) die Verbesserung der Elastizität oder Reduzierung von Hautfalten, oder zur Behandlung oder Verhinderung von (ii) Hypertonie, (iii) Retinopathie, (iv) Schäden der Linsenproteine, (v) Katarakte, (vi) periphere Neuropathien oder (vii) Osteoarthritis.

3. Verbindung wie in Formel (I) definiert oder ein biologisch oder pharmazeutisch verträgliches Säureadditionssalz davon zur Verwendung für die Behandlung von Diabetes oder zur Behandlung oder Verhinderung nachteiliger Folgeerscheinungen von Diabetes.

4. Verbindung wie in Formel (I) definiert oder ein biologisch oder pharmazeutisch verträgliches Säureadditionssalz davon zur Verwendung für die Behandlung oder Verhinderung von Nierenschäden.

5. Verbindung wie in Formel (I) definiert oder ein biologisch oder pharmazeutisch verträgliches Säureadditionssalz davon zur Verwendung für die Behandlung oder Verhinderung von Blutgefäßen.

6. Verbindung nach einem der Ansprüche 1 bis 5, wobei R eine Methylgruppe ist.

7. Verbindung nach einem der Ansprüche 1 bis 5, welche N-(2-acetamidoethyl)hydrazincarboximidamidhydrobromid, N-(2-acetamidoethyl)hydrazincarboximidamidhydrochlorid, N-(3-acetamidopropyl)hydrazincarboximidamidhydrobromid oder ein anderes pharmazeutisch verträgliches Salz davon ist.

8. Verbindung nach Anspruch 7, welche N-(2-acetamido-ethyl)-hydrazincarboximidamidhydrochlorid oder N-(2-acetamidoethyl)hydrazincarboximidamidhydrochloridhydrobromid ist, zur Verwendung für die Behandlung eines Tieres zur Inhibierung der Bildung von fortgeschritten glykosilierter Endprodukte eines Zielproteins in dem Tier ohne begleitende Wirkungen auf die Enzyme Diaminoxidase und induzierbarer Stickstoffoxidsynthase.

9. Pharmazeutische Zusammensetzung zur Verabreichung an ein Tier zur Inhibierung der fortschreitenden Glykosilierung eines Zielproteins in dem Tier, umfassend eine pharmazeutisch wirksame Menge einer Verbindung wie in einem der Ansprüche 1 bis 8 definiert zusammen mit einem Träger dafür.

10. Pharmazeutische Zusammensetzung nach Anspruch 9 zur Verwendung für die Behandlung eines Tieres zur Inhibierung der Bildung von fortgeschrittenen Glykosilierungsendprodukten eines Zielproteins in dem Tier.

11. Pharmazeutische Zusammensetzung zur Verwendung für (i) die Verbesserung der Elastizität oder Reduzierung von Hautfalten, zur Behandlung (ii) von Diabetes oder zur Behandlung oder Verhinderung (iii) nachteiliger Folgeerscheinungen von Diabetes, (iv) Nierenschäden, (v) Schäden der Blutgefäße, (vi) Hypertension, (vii) Retinopathie, (viii) Schäden der Linsenproteine, (ix) Katarakte, (x) periphere Neuropathien oder (xi) Osteoarthritis, umfassend eine pharmazeutisch wirksame Menge einer Verbindung wie in einem der Ansprüche 1 bis 8 definiert zusammen mit einem Träger dafür.

12. Verbindung nach einem der Ansprüche 1 bis 8 oder eine Zusammensetzung nach Anspruch 9 zur Verwendung für die Inhibierung der Verfärbung von Zähnen als Folge von nicht-enzymatischer Bräunung in der Mundhöhle.

**Revendications**

1. Composé de formule (1)

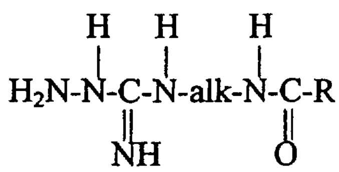

(I)

dans laquelle

alk est un groupe alkylène à chaîne linéaire ou ramifiée contenant de 2 à 8 atomes de carbone, et
R est un groupe alkyle inférieur contenant de 1 à 6 atomes de carbone;
ou
un sel d'addition d'acide acceptable en biologie ou en pharmacie de ce composé,

pour inhiber la glycosylation avancée d'une protéine cible.

2. Composé tel que défini dans la formule (1) ou sel d'addition d'acide acceptable en biologie ou en pharmacie de ce composé à utiliser (i) pour améliorer l'élasticité ou réduire les rides de la peau, ou bien pour traiter ou prévenir (ii) (hypertension, (iii) la rétinopathie, (iv) les lésions aux protéines du cristallin, (v) la cataracte, (vi) la neuropathie périphérique ou (vii) l'arthrose.

3. Composé tel que défini dans la formule (1) ou sel d'addition d'acide acceptable en biologie ou en pharmacie de ce composé à utiliser pour traiter le diabète, ou bien pour traiter ou prévenir les séquelles du diabète.

4. Composé tel que défini dans la formule (1) ou sel d'addition d'acide acceptable en biologie ou en pharmacie de ce composé à utiliser pour traiter ou prévenir les lésions rénales.

5. Composé tel que défini dans la formule (I) ou sel d'addition d'acide acceptable en biologie ou en pharmacie de ce composé, pour traiter ou prévenir les troubles du système sanguin vasculaire.

6. Composé selon l'une quelconque des revendications 1-5 dans lequel R est un groupe méthyle.

7. Composé selon l'une quelconque des revendications 1-5 qui est:

le bromhydrate de N-(2-acétamidoéthyl)hydrazinecarboximidamide;
le chlorhydrate de N-(2-acétamidoéthyl)hydrazinecarboximidamide;
le bromhydrate de N-(3-acétamidopropyl)hydrazinecarboxirnidamide; ou

un autre sel de ces composés acceptables en pharmacie.

8. Composé selon la revendication 7 qui est

le chlorhydrate de N-(2-acétamidoéthyl)hydrazinecarboximidamide ou
le chlorhydrate/bromhydrate de N-(2-acétamidoéthyl)hydrazinecarboximidamide

à utiliser pour traiter un animal pour inhiber la formation de produits finals de glycosylation avancée d'une protéine cible chez ledit animal, sans effets concomitants sur les enzymes diaminooxydase et la forme inductible de l'oxyde nitrique synthase.

9. Composition pharmaceutique destinée à l'administration à un animal pour inhiber la glycosylation avancée d'une protéine cible chez ledit animal, comprenant une quantité efficace sur le plan pharmaceutique d'un composé selon l'une quelconque des revendications 1 à 8 ainsi qu'un véhicule pour ce composé.

**10.** Composition pharmaceutique selon la revendication 9, à utiliser pour traiter un animal pour inhiber la formation de produits finals de glycosylation avancée d'une protéine cible chez ledit animal.

**11.** Composition pharmaceutique à utiliser (i) pour améliorer l'élasticité ou réduire les rides de la peau, ou bien pour traiter (ii) le diabète ou bien pour traiter ou prévenir (iii) les séquelles néfastes du diabète, (iv) les lésions rénales, (v) les troubles du système sanguin vasculaire, (vi) l'hypertension, (vii) la rétinopathie, (viii) les lésions aux protéines du cristallin, (ix) la cataracte, (x) la neuropathie périphérique ou (xi) l'arthrose, comprenant une quantité efficace sur le plan pharmaceutique d'un composé selon l'une quelconque des revendications 1 à 8, ainsi qu'un véhicule pour ledit composé.

**12.** Composé selon l'une quelconque des revendications 1 à 8 ou composition selon la revendication 9 à utiliser pour inhiber la coloration des dents résultant d'un brunissement non enzymatique dans la cavité buccale.